# EUROPEAN PATENT APPLICATION

(11) **EP 2 982 745 A1**
(43) Date of publication of application: **10.02.2016**
(21) Application number: 14179989.0
(22) Date of filing: 06.08.2014
(51) Int. Cl.: C12N 5/078, A61K 35/14, C12N 5/0783

(54) **Method for expanding immune cells**

(71) Applicant: Johann Wolfgang Goethe-Universität, Frankfurt am Main, 60323 Frankfurt am Main (DE)
(72) Inventor: Ullrich, Evelyn, 60594 Frankfurt (DE); Gaetano, Carlo, 60598 Frankfurt am Main (DE); Worm, Catharina, 65347 Eltville-Hattenheim (DE); Cencioni, Chiara, 60385 Frankfurt am Main (DE)
(74) Representative: Krauss, Jan

(57) **Abstract**

The present invention pertains to a method for the *in-vitro* or *ex-vivo* expansion of immune cells using a histone acetyl transferase (HAT) modulator as additive in the cell culture medium during the expansion procedure. Immune cells such as natural killer (NK) cells, T cells or cytokine induced killer (CIK) cells are used in adoptive cell therapies for the injection into patients suffering from malignant cancer diseases such as leukemia. Cells expanded using the method of the invention were superior proliferative and viable. Further provided by the invention is a cell culture medium comprising the HAT modulator of the invention as well as cell populations obtained using the method of the invention.

## Description

### FIELD OF THE INVENTION

The present invention pertains to a method for the *in-vitro* or *ex-vivo* expansion of immune cells using a histone acetyl transferase (HAT) modulator as additive in the cell culture medium during the expansion procedure. Immune cells such as natural killer (NK) cells, T cells or cytokine induced killer (CIK) cells are used in adoptive cell therapies for the injection into patients suffering from malignant cancer diseases such as leukemia. Cells expanded using the method of the invention were superior proliferative and viable. Further provided by the invention is a cell culture medium comprising the HAT modulator of the invention as well as cell populations obtained using the method of the invention.

### DESCRIPTION

Adoptive immune cell transfer or therapy is a method wherein immune-derived cells are transferred into a patient suffering from a severe disorder such as leukemia, in order to support the patient's immune system. The immune cells to be transferred may be derived from the patient themselves (autologous cells), which are subsequent to purification and leukapheresis *in-vitro* expanded in order to increase activity and cell numbers before the reinjection. Alternatively, the immune cells are derived from a donor (allogeneic cells). For example in the case of a patient who received a hematopoietic stem cell transplantation, immune cells for adoptive cell therapy are ideally derived from the same donor who provided the stem cell sample for the transplantation. High numbers of viable and active cells are imperative for an effective immune cell therapy. To this end the obtained immune cell sample is usually purified to obtain a pure population of the desired cell type, before they are *in-vitro* or *ex-vivo* expanded to obtain sufficient cell numbers for the transfer.

Primary non-adherent cells such as peripheral blood mononuclear cells (PBMCs), T cells, natural killer cells (NK cells), cytokine induced killer (CIK) cells, regulatory T cells (Treg), bone marrow derived cells (including MSCs and MDSCs), stem cells and islets are often the focus of *ex-vivo* cell expansion. Many production processes aim to increase the population of desired cells, often referred to as effector cells, often in co-culture conditions that rely on other cell types to stimulate growth and/or antigen specificity of the desired cells. In some cases, co-cultures transition to expansion of the desired cell population in the absence of feeder and/or antigen presenting cells such as TIL production. Production of antigen presenting cells and/or feeder cells in the absence of effector cells is also prevalent. Also, sometimes culture is intended to maintain health of a cell population as opposed to increasing the population per se, such as islet culture for treatment of diabetes.

Recent studies have emphasized the potential of NK cell therapy in recipients of allogeneic hematopoietic stem cell transplantation. In animal models of transplantation, donor NK cells could lyse leukemic cells and host lympho-hematopoietic cells without affecting nonhematopoietic tissues (Caligiuri M A, Velardi A, Scheinberg D A, Borrello I M. Immunotherapeutic approaches for hematologic malignancies. Hematology; Am Soc Hematol Educ Program 337-353 (2004)), thereby demonstrating NK-mediated graft-versus-leukemia responses. Because NK cells are inhibited by self-HLA molecules which bind to killer immunoglobulin-like receptors (KIR), these findings have led to clinical cell therapeutic protocols selecting hematopoietic stem cell transplant donors with an HLA and KIR type that favors NK cell activation and thus could be expected to promote an antileukemic effect (Sadelain M, Riviere I, Brentjens R. Targeting tumours with genetically enhanced T lymphocytes. Nat Rev Cancer 3:35-45 (2003); Cooper L J, Topp M S, Serrano L M, et al. T cell clones can be rendered specific for CD 19: toward the selective augmentation of the graft-versus-B-lineage leukemia effect. Blood 101:1637-1644 (2003); Brentjens R J, Latouche J B, Santos E, et al. Eradication of systemic B-cell tumors by genetically targeted human T lymphocytes co-stimulated by CD80 and interleukin-15. Nat Med 9:279-286 (2003)). However, selection of the "best" donor is limited to patients who have more than one potential donor and the capacity of NK cells to lyse lymphoid cells is generally low and difficult to predict (Sadelain M, Riviere 1, Brentjens R. Targeting tumours with genetically enhanced T lymphocytes. Nat Rev Cancer 3:35-45 (2003); Brentjens R J, Latouche J B, Santos E, et al. Eradication of systemic B-cell tumors by genetically targeted human T lymphocytes co-stimulated by CD80 and interleukin-15. Nat Med 9:279-286 (2003); Imai C, Mihara K, Andreansky M, Nicholson I C, Pui C H, Campana D. Chimeric receptors with 4-1BB signaling capacity provoke potent cytotoxicity against acute lymphoblastic leukemia. Leukemia 18:676-684 (2004); Ito C, Kumagai M, Manabe A, et al. Hyperdiploid acute lymphoblastic leukemia with 51 to 65 chromosomes: A distinct biological entity with a marked propensity to undergo apoptosis. Blood 93:315-320 (1999)). The established methods for cell expansion favor T cell expansion and even after T cells are depleted, residual T cells typically become prominent after stimulation. Methods for the expansion of T lymphocytes have been described in US published patent applications no. 20030147869, 20030224520, 20040101519 and 20040110290.

Current methods for the expansion of immune cells include the *ex-vivo* culturing of purified populations in the presence of cytokines, such as Interleukin (IL)-2, IL-12, IL-15, IL-18, IL-21, or Interferon (IFN) type I. NK cell purification and expansion protocols using IL-2 and IL-15 were developed for activating allogeneic NK cells (such as Koehl U, et al. 2013, *Frontiers in Oncology*). However, immunotherapy with NK cells still is limited by the inability to obtain sufficient numbers of pure NK cell populations suitable for manipulation and expansion. Thus there is a need for better methods to preferentially expand NK cells.

The acetylation and deacetylation of histones plays a key role in the regulation of gene expression in eukaryotic cells. The acetylation status of histones alters chromatin structure, and thereby modulates gene expression. Two classes of enzymes can affect the acetylation of histones: histone acetyltransferases (HATs) and histone deacetylases (HDACs). Interestingly, these enzymes can also acetylate or deacetylate several non-histone substrates with functional consequences. Altered HAT and HDAC activities can lead to several diseases, ranging from cancer to neurodegenerative diseases.

Several families of HATs have recently been identified, which includes, GNAT family (GCN 5 - related N - acetyltransferase), the MYST group, SAS2, TIP60, and p300/CBP families. The p300/CBP family of HAT is represented by two of the most widely studied HATs, p300 and CBP. These proteins share considerable sequence and functional homology. Several lines of evidence indicate that p300/CBP are involved in cell cycle progression and cellular differentiation. Mechanistically, these proteins function as transcriptional co-activators through their direct interaction with a diverse group of transcription factors and the RNA polymerase II transcription machinery. The co-activation function is partially facilitated by their intrinsic HAT activity.

The p300/CBP associated factor (PCAF) is one of the important HATs of the GNAT family. The C-terminal half of PCAF has a very significant sequence similarity to yeast GCN5. In humans there are two GCN5 splice variants, hGCN5 and hGCN5-L (long form) synthesized from the same gene. The hGCN5 - L is similar in length to PCAF and shares 75% amino acid sequence identity with PCAF. It also interacts with p300/CBP. In vivo PCAF exists in a large multi-protein complex, containing more than 20 different polypeptides. Unlike p300/CBP (which acetylates all the four core histones, predominantly H3 and H4) PCAF acetylates predominantly histone H3.

In view of the above described limits of adoptive immune cell therapy, the present invention seeks to increase efficiency of adoptive immune cell therapy by improving *ex-vivo* immune cell expansion.

The above problem is solved in a first aspect by a method for the production of or proliferation of immune cells, the method comprising the step of culturing an immune cell in the presence of an histone acetyl transferase (HAT)-modulator.

The method according to the invention is in a preferred embodiment an *ex-vivo* or *in-vitro* method.

Additionally the method may include a step of isolating the cells as expanded. Also the method may include a preparation step wherein before culturing the cells in the presence of said HAT modulator, the immune cells are purified (a purification step).

In context of the present invention it was surprisingly found that the addition of the HAT-modulator SPV-106 to the culture medium during the *ex-vivo* expansion of immune cells such as T cells, NK cells or CIK cells, leads to enhanced cell proliferation. The cells expanded in accordance with the method of the invention showed superior viability, proliferative capacity, maintenance of phenotype, subset distribution and cytotoxic anti-tumoral potential.

The term "modulator" in context of the present invention either refers to an antagonist and/or agonist of a HAT. A modulator is therefore any substance that can antagonise or agonise the expression, activity, function or stability of a given HAT. Mixed agonists and antagonists of HATs are well known in the art.

In one preferred embodiment of the invention said HAT-modulator is a p300/CREB binding protein (CBP)-antagonist and/or a p300/CBP-binding protein associated factor (PCAF)-agonist.

HAT modulators that are preferred in context of the herein described invention are selected from the group consisting of SPV-106, Lys-CoA, H3-CoA-20, garcinol, LTK-14, curcumin, isothiazolones, anarcardic acid, MB-3, CTBP, MC1823.

Most preferred in context of the present invention is that the HAT-modulator is a p300/CREB binding protein (CBP)-antagonist and a p300/CBP-binding protein associated factor (PCAF)-agonist. SPV-106 is such a mixed HAT modulator and has the formula (I). Included are also derivatives or salts of this compound.

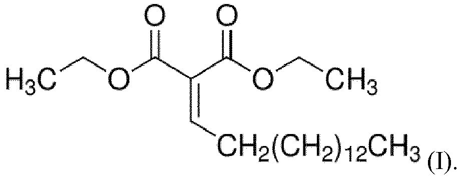

Alternatively preferred is the inhibitor N-[4-chlor-3-trifluoromethylphenyl]-2-ethoxy-6-pentadecyl-benzamide (CTPB), or derivatives or salts of this compound.

In the context of the present invention an "immune cell" is preferably selected from the group of blood leucocytes, preferably a natural killer (NK)-cell, a cytokine induced killer (CIK)-cell or a T cell. Preferred in the context of the invention is the use of natural killer cells as immune cells.

The term "natural killer cells" or "NK cells" is meant to include any cells and/or their products which are equivalent to natural killer cells and/or their products in the sense that they likewise have the effect of suppressing tumor growth of NK-sensitive cells *in situ.* NK cells are cytotoxic lymphocytes which lack the phenotypic markers of both T cells (TCR, CD3) and B-cells (membrane immunoglobulin). They bear CD16 and CD56 as characteristic markers, and are characterized by the absence of CD3. NK cells can therefore alternatively be denoted as CD56⁺CD3⁻ cells. They contain prominent cytoplasmic granules and are morphologically distinguishable as "large granular lymphocytes," and the granules contain perforine, granzymes, cytokines, chemokines, regulatory proteins (such as CD95L) and multiple other cell release which are inserted into the membranes of target cells. Activated NK cells make various cytokines. A heterodimeric cytokine IL-12 activates NK as well as T cells. The products of NK cells include, but are not limited to, interferon gamma (IFN-y), CD95L, and CD154 (ligand of CD40). Natural killer cells are further divided into two sub-groups depending on the expression level of the surface markers CD56 and CD16. One group of natural killer cells is characterized by a low CD56 expression and a high CD16 expression (about 90% of all NK cells), whereas the other group is characterized by a high CD56 expression and a low CD16 expression. Therefore, in one embodiment of the invention it is preferred that said immune cell is a CD56^{dim}CD16^{high} NK cell or a CD56^{high}CD16^{dim} NK cell.

The immune cell that is used in accordance with the various embodiments or aspects of the invention is preferably a vertebrate cell, more preferably a mammalian cell, such as a mouse, rat, monkey, or most preferably, a human cell. A human cell is preferably an autologous immune cell obtained from a patient suffering from a cancer disease. Human embryonic stem cells are excluded from the scope of the invention if they are obtainable exclusively by a method comprising the destruction of human embryos. The method of the invention allows for an expansion of the autologous cell. The expanded autologous cell population can then be used for injection during a therapy of said patient.

Alternatively said immune cell is obtained from a donor of a hematopoietic stem cell transplantation, or from an allogeneic individual of said donor. A haploidentical individual is closely related to the donor of the stem cells. In this embodiment it is intended that the expanded cell population derived from a sample of a donor of a hematopoietic stem cell-transplantation are used to treat the patient that before received said stem cells. These cells, as also described above, are allogeneic cells.

In one additional embodiment a method in accordance of the invention includes that said immune cell is cultured in the presence of at least one or more additional compounds selected from the group of cytokines, in particular Interleukin (IL)-2, IL-12, IL-15, IL-18, IL-21, or Interferon (IFN) type I. Particularly preferred is the additional presence of IL-2, most preferred the additional presence of IL-2 and IL-15.

In a preferred application of the method of the present invention said HAT-modulator is present in a concentration of between 0.1 to 1000 µM, preferably of between 1 to 100 µM, more preferably of between 1 to 50 µM, more preferably of 1 to 10 µM, most preferably of about 5 µM.

The above problem is furthermore solved by providing a HAT-modulator for use in the treatment of a patient suffering from a cancer disease, wherein said treatment comprises a method for adoptive immune cell transfer. Such an adoptive immune cell transfer is particularly useful after allogeneic hematopoietic stem cell transplantation (HSCT).

In one embodiment of the above aspect, said method for adoptive immune cell transfer comprises the steps of
a. Providing an immune cell sample from a donor,
b. Expanding the immune cells of said sample in the presence of said HAT-modulator, and
c. Administering the expanded immune cells of step (b) to said patient suffering from a malignant disease.

Preferably said immune cell is selected from the group of blood leucocytes, preferably a natural killer (NK) cell, a cytokine induced killer (CIK) cell or a T cell, most preferably a CD56^{dim}CD16^{high} NK cell or a CD56^{high}CD16^{dim} NK cell. These cells are preferably of mammalian origin, most preferably of human origin.

In one preferred embodiment of the invention said immune cell is a natural killer cell. In this context it is optional that after obtaining a cell sample from said donor, said donor cell sample containing a mixture of immune cells is purified in advance to the cell expansion step. For adoptive NK cell therapy the purification comprises a step of separating T cells from the NK cells. This is advantageous in order to increase proliferation of the NK cell fraction which is later used for the administration to a patient. Such purification steps of donor samples are known to the skilled person, for example from Koehl U, et al., *Frontiers in Oncology,* 2013.

In context of the present invention said donor and said patient is the same individual. In this embodiment the adoptive immune cell transfer would be an adoptive autologous immune cell transfer. Alternatively, the donor can be a different individual.

Alternatively said patient that will be subject to the adoptive immune cell transfer received an allogeneic hematopoietic stem cell-transplantation in advance. In this case said donor of said immune cells is preferably the donor of said hematopoietic stem cells, or an individual that is haploidentical to said donor of said hematopoietic stem cells.

Further preferred is that the expansion of said immune cells during the treatment in step (b) is performed *ex-vivo* or *in-vitro.*

One additional embodiment that may be preferred relates to the above HAT-modulator, wherein said immune cells in step (b) are expanded in the presence of at least one or more additional compounds selected from the group of cytokines, in particular Interleukin (IL)-2, IL-12, IL-15, IL-18, IL-21, or Interferon (IFN) type I.

The terms "cancer disease" and "malignant disease" are used synonymously herein and include not only carcinomas of soft and hard tissues and blood carcinomas; they also extends to skin, tissue, organ, bone, cartilage, blood and vessel diseases. The terms "cancer disease" and "malignant disease" further include primary and metastasizing types of cancerous proliferation.

Particularly preferred types of cancers for which the cell transfer therapies of the present invention will be advantageous are selected from the group consisting of solid or hematopoietic tumors e.g adenocarcinoma, hypopharynx cancer, lung cancer, diffuse large cell lymphoma, Burkitt's lymphoma, Hodgkin lymphoma, Non-Hodgkin lymphoma, histiocytic lymphoma, lymphatic lymphoma, acute T cell leukemia, pre-B- acute lymphoblastic leukemia, chronic and acute myelogenous leukemia (CML), gastrointestinal cancers eg.(gall bladder-, stomach-, esophageal-, pancreatic-, colon cancer, bile duct carcinoma) thymus carcinoma, urothelium carcinoma, testicular cancer, prostate cancer, bladder cancer, brain tumour, skin tumor including AIDS-related Kaposi's sarcoma, Ewing sarcoma, rhabdomyosarcoma, neuroblastoma, ovarian cancer, head and neck cancer, osteosarcoma, melanoma, breast cancer and CUP syndrome.

The term "patient" in context of the present invention refers to a subject suffering from a disease as defined herein above, preferably a human patient. In particular the patient suffers from a cancer disease in a late stage, such as a late diagnosed cancer or a relapsed or recurrent cancer. Also, a patient may have already received allogeneic hematopoietic stem cell transplantation.

A donor in the context of the herein described invention is either the patient. This is the case for autologous immune cell transfers. Alternatively, the donor is a different and healthy individual, preferably an individual that is closely related to the patient. In the case where the patient received allogeneic hematopoietic stem cell transplantation, the donor is preferably the same individual that provided the hematopoietic stem cells.

The HAT-modulator is preferably a HAT modulator as defined herein above.

In an alternative aspect there is also provided a method for the treatment of a patient suffering from a cancer disease, said method comprising the steps of providing an immune cell sample from a donor, expanding immune cells of said sample in the presence of an effective amount of said HAT-modulator (as described herein before), and administering an effective amount of the expanded immune cells to said patient suffering from said cancer disease. The above and below described preferred embodiments equally apply for the method of treatment of the present invention.

There is furthermore provided a solution to the aforementioned problem which is a use of a HAT-modulator, preferably as defined herein above, in the maintenance, culturing, expansion or proliferation of an immune cell. The use in accordance of the invention preferably includes a maintenance, culturing, expansion or proliferation of an immune cell that is performed *in-vitro* or *ex-vivo.* Preferred is that the use is a use of the HAT-modulator as an additive or supplement in a cell culture medium.

Yet another aspect of the invention pertains to a population of cells as produced according to the method for the production or expansion of immune cells as described in detail herein above.

Additionally the above problem in the prior art is also solved by a cell culture medium comprising a HAT-modulator as defined herein above.

The terms, "cell culture medium," "culture medium," or "medium" (and in each case plural media) as used in this application refer to a nutritive composition that supports the cultivation and/or growth of cells. The cell culture medium may be a complete formulation, i.e., a cell culture medium that requires no supplementation to culture cells, may be an incomplete formulation, i.e., a cell culture medium that requires supplementation or may be a medium that may supplement an incomplete formulation or in the case of a complete formulation, may improve culture or culture results. The terms "cell culture medium," "culture medium," or "medium" (and in each case plural media) refer both to unconditioned cell culture media that has not been incubated with cells, or to "spent" or "conditioned" medium, which may contain many of the original components of the medium, as well as a variety of cellular metabolites and secreted proteins.

In one embodiment the cell culture medium of the invention may further comprise at least one or more additional compounds selected from the group of cytokines, in particular Interleukin (IL)-2, IL-12, IL-15, IL-18, IL-21, or Interferon (IFN) type I. Preferred is IL-2 as additional additive; most preferred is IL-2 and IL-15.

Preferred is that the cell culture medium of the invention is suitable for culturing mammalian, preferably human cells, such as human leucocytes. The basic ingredients of cell culture media used for culturing for example human blood cells are well known to the person of skill in the art. Preferably the cell culture medium according to the invention is suitable for culturing an immune cell, most preferably a T cell, CIK cell or an NK cell. Also media suitable for these purposes are known to the skilled person -as exemplified in the example section below.

The present invention will now be further described in the following examples with reference to the accompanying figures and sequences, nevertheless, without being limited thereto. For the purposes of the present invention, all references as cited herein are incorporated by reference in their entireties. In the Figures:
- **Figure 1:**: Expansion of the NKL cell line by stimulation with a combination of cytokines and SPV-106
- **Figure 2:**: Expansion of primary NK cells by combination of cytokines and SPV-106
- **Figure 3:**: NK cell subset distribution during NK cell expansion
- **Figure 4:**: Cytotoxicity of NK cells expanded by cytokines and SPV106

### EXAMPLES

### Materials and Methods

### Cell culture

NKL cell line was grown in Iscove's Modified Dulbecco's Medium (IMDM-Lonza) supplemented with 20% fetal bovine serum and recombinant human interleukine 2 (IL-2, 100 U/ml). Proliferation assay was performed by culturing 2x10⁵ NKL cells/ml (96 multiwell; Nunc Thermo Scientific) in 200 µl growth medium supplemented with IL-2 (100 U/ml); IL-2/-15 (10 ng/ml); or IL-2/-15/SPV-106 5µM. Cells were counted and culture medium was changed every three days. Primary NK cells were isolated from peripheral blood of healthy donors using different methods of NK cell isolation (such as *Easy Sep Human NK Cell Enrichment Kit, Stem cell Technologies* or *Human NK Cell Negative Selection Kit, Miltenyi*)*.* Proliferation assay was performed by culturing 2x10⁶ primary NK cells/ml (24 multiwell ) in 1 ml growth medium supplemented with IL-2 (100 U/ml)/IL-15 (10 ng/ml); or IL-2/-15/SPV-106 5µM. Cells were counted and culture medium was changed every three days. Average of relative cell counts was determined microscopically using Trypan blue staining to exclude death or damaged cells.

### Cytotoxicity assay

Effector cells were generated prepared as described above. As target cells, K562, a human chronic myelogenous leukemia cell line, derived from an acute monocytic leukemia patient, were maintained in culture in RPMI 1640 medium supplemented with 10% fetal bovine serum. The medium was changed three times a week, and the cells were in the log phase of growth when used as targets in cytotoxicity assays. About 5 x10⁶ target cells were washed in saline to reduce extracellular Ca⁺² content. The cell pellet was resuspended in 1 ml of labeling buffer supplemented with 20 mM Eu(DH3C00)³⁺, 100 mM DTPA, and 0.5 mg of dextran sulfate. This cell suspension was incubated for 20 min at room temperature with occasional shaking. The labeling process was stopped by the addition of 30 ml of 100 mM CaCl₂ solution per ml. After 5 min of continued incubation, the cells were washed three times in 30 ml of repair buffer and then one to two times in 10 ml of medium and were finally resuspended in CM at a concentration of 5 x 10⁴ cells/ml. Target cells labeled with Eu31 were adjusted to a concentration of 5 x 10³ cells/100 ml in complete medium. Aliquots (100 ml) of target cells were dispensed into wells of 96-well round-bottomed microtiter plates. An equal volume of effector cells was added to each well. Suspensions of effector cells were adjusted to give effector/target (E/T) ratios of 2.5:1 or 5:1 or 10:1 for NK activity. In all assays, three different E/T ratios were used. The microplates were centrifuged briefly to bring effectors and targets in contact with each other and then incubated for 4 h at 37°C in a humidified atmosphere of 5% CO2 in air. All assays were done in triplicate. After incubation, the plates were centrifuged again, and the supernatants were harvested for measurements of released Eu31 by in a time-resolved fluorometer (Victor 1420-018, Perkin Elmer, MA, USA). The percentage of specific cytotoxicity was calculated as follows: ((experimental release (counts)-spontaneous release (counts)) : (maximal release (counts)-spontaneous release (counts)) x 100.

### Example 1: Expansion of an NKL Cell Line with SPV-106

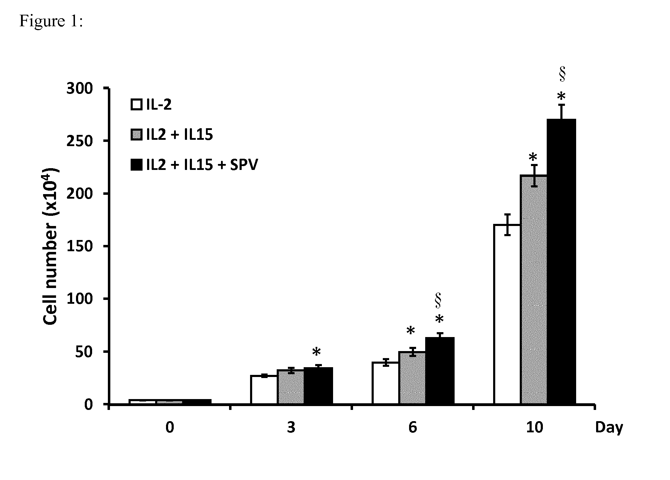

NKL cells were cultured in growth medium for 10 days in presence of IL-2, IL-2/-15, or IL-2/-15/SPV-106 (5µM). SPV-106 is the selective activator of histone acetylase PCAF. At 3, 6 and 10 days cells were counted. Average cell number (x 10⁴) is shown in the graph of figure 1 (n = 7 at each time point). SPV-106 enhances NKL cell line proliferation already after 3 days of culture. Statistical significance: * p< 0.05 vs IL-2 cells and § p<0,005 vs IL-2 + IL-15 cells.

### Example 2: Expansion of Primary NK Cells with SPV-106

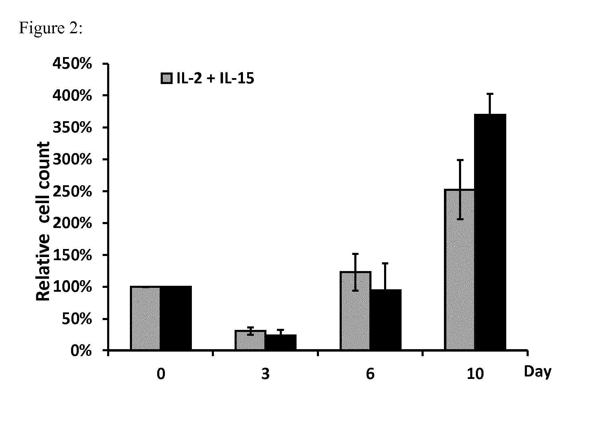

Primary NK cells isolated from peripheral blood were cultured in growth medium for 10 days in presence of IL-2/-15, or IL-2/-15/SPV-106 (5µM). At 3, 6 and 10 days cells were counted. Average of relative cell counts in comparison of 100% at day 0 is shown in the graph of figure 2 (n = 3 at each time point). SPV-106 enhances primary NK cell proliferation at 5 µM after 10 days of culture.

### Example 3: NK cell subset distribution during NK cell expansion

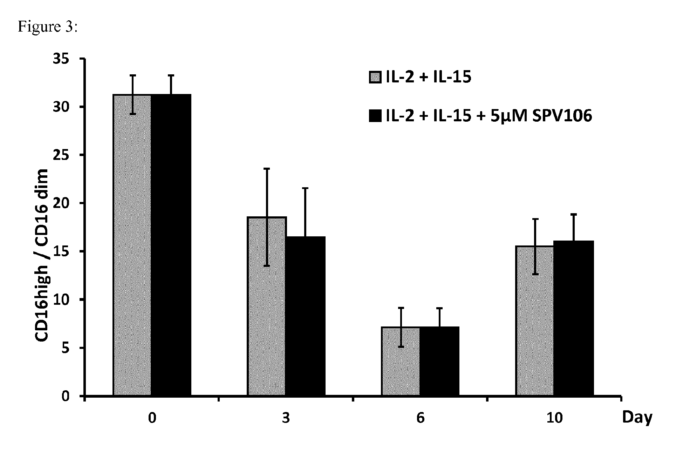

Primary NK cells were cultured in growth medium for 10 days in presence of IL-2/-15, IL 2/-15, or IL-2/-15/ SPV-106 (at 5µM). At 3, 6 and 10 the NK cell subset distribution (CD56^{high} : CD56^{dim}) was analysed by flow cytometry. Average of the NK cell subset ratio is shown in the graph of figure 3 (n = 3 at each time point). SPV-106 did not change the naturally occurring NK subset distribution.

### Example 4: Cytotoxicity of SPV-106

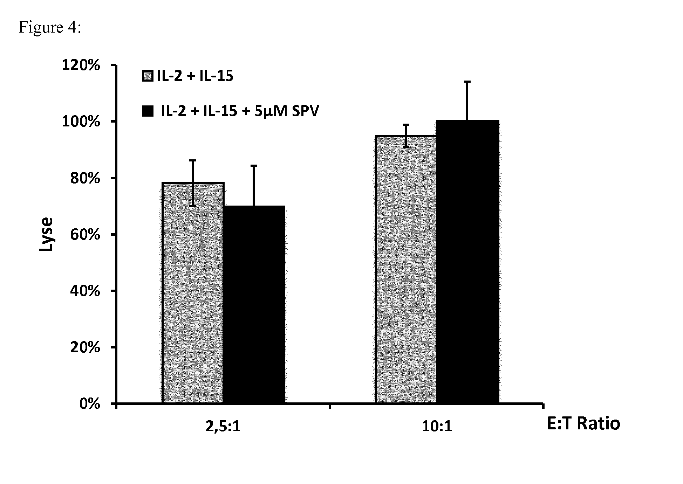

NK cells that have been expanded by cytokines and 5µM SPV-106 showed retained cytotoxic capacity as shown in figure 4. Killing activity of K562 target cells was assessed by Europium Assay as described in the following Material and Methods section.

## Claims

1. A method for the production or proliferation of immune cells, the method comprising the step of culturing an immune cell in the presence of an histone acetyl transferase(HAT)-modulator.

2. The method according to claim 1, wherein said immune cell is selected from the group of blood leucocytes, preferably a natural killer (NK)-cell, a cytokine induced killer (CIK)-cell or a T cell.

3. The method according to any of the preceding claims, wherein said immune cell is further cultured in the presence of at least one or more additional compounds selected from the group of cytokines, in particular Interleukin (IL)-2, IL-12, IL-15, IL-18, IL-21, IL-5 or Interferon (INF) type I.

4. The method according to any of the preceding claims, wherein said HAT-modulator is a p300/CREB binding protein(CBP)-antagonist and/or a p300/CBP-binding protein associated factor (PCAF)-agonist.

5. The method according to any of the preceding claims, wherein said HAT-modulator is selected from the group of compounds consisting of SPV-106, Lys-CoA, H3-CoA-20, garcinol, LTK-14, curcumin, isothiazolones, anarcardic acid, MB-3, CTBP, MC1823, preferably said HAT-modulator is compound having the formula (I) 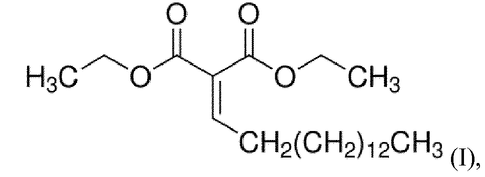 or
N-[4-chlor-3-trifluoromethylphenyl]-2-ethoxy-6-pentadecyl-benzamide (CTPB), or derivatives or salts of these compounds.

6. A HAT-modulator for use in the treatment of a cancer disease, wherein said treatment comprises a method for adoptive immune cell transfer.

7. The HAT-modulator according to claim 6, wherein said cancer disease is selected from the group consisting of solid or hematopoietic tumors e.g adenocarci-noma, hypopharynx cancer, lung cancer, diffuse large cell lymphoma, Burkitt's lymphoma, Hodgkin lymphoma, Non-Hodgkin lymphoma, histiocytic lymphoma, lymphatic lymphoma, acute T cell leukemia, pre-B cell acute lymphoblastic leukemia (B-ALL), chronic and acure myeloitic leukemia, Gastrointestinal cancers eg.(gall bladder-, stomach-, esophageal-, pancreatic-, colon cancer, bile duct carcinoma) thymus carcinoma, urothelium carcinoma, testicular cancer, prostate cancer, bladder cancer, brain tumour, skin tumor including AIDS-related Kaposi's sarcoma, Ewing sarcoma, rhabdomyosarcoma, neuroblastoma, ovarian cancer, head and neck cancer, osteosarcoma, melanoma, breast cancer and CUP syndrome.

8. The HAT-modulator according to claim 6 or 7, wherein said HAT modulator is a p300/CREB binding protein(CBP)-antagonist and/or a p300/CBP-binding protein associated factor (PCAF)-agonist.

9. The HAT-modulator according to any of claims 6 to 8, wherein said HAT-modulator is selected from the group of compounds consisting of SPV-106, Lys-CoA, H3-CoA-20, garcinol, LTK-14, curcumin, isothiazolones, anarcardic acid, MB-3, CTBP, MC1823, preferably said HAT-modulator is compound having the formula (I) 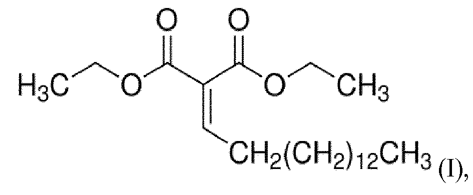 or N-[4-chlor-3-trifluoromethylphenyl]-2-ethoxy-6-pentadecyl-benzamide (CTPB), or derivatives or salts of these compounds.

10. *In-vitro* use of a HAT-modulator in the maintenance, culturing, expansion or proliferation of an immune cell.

11. The use according to claim 10, wherein said HAT modulator is a p300/CREB binding protein(CBP)-antagonist and/or a p300/CBP-binding protein associated factor (PCAF)-agonist.

12. The use according to claim 10 or 11, wherein said HAT-modulator is selected from the group of compounds consisting of SPV-106, Lys-CoA, H3-CoA-20, garcinol, LTK-14, curcumin, isothiazolones, anarcardic acid, MB-3, CTBP, MC1823, preferably said HAT-modulator is compound having the formula (I) 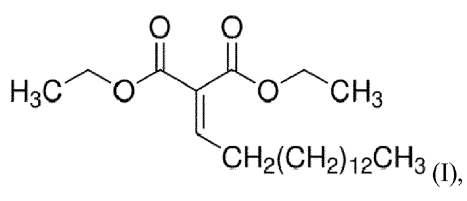 or N-[4-chlor-3-trifluoromethylphenyl]-2-ethoxy-6-pentadecyl-benzamide (CTPB), or derivatives or salts of these compounds.

13. A cell culture medium comprising a HAT-modulator as defined according to any of the preceding claims.

14. The cell culture medium according to claim 13, further comprising at least one or more additional compounds selected from the group of cytokines, in particular Interleukin(IL)-2, IL-12, IL-15, IL-18, IL-21, IL-5 or Interferon (INF) type I.

15. The cell culture medium according to claim 13 or 14, which is suitable for culturing an immune cell, most preferably a T cell, CIK cell or an NK cell.
